# EUROPEAN PATENT SPECIFICATION

(11) **EP 2 258 333 B1**
(45) Date of publication and mention of the grant of the patent: **29.08.2012**
(21) Application number: 09007303.2
(22) Date of filing: 02.06.2009
(51) Int. Cl.: A61J 1/20, B65B 3/00

(54) **Device for filling a flexible reservoir**
Vorrichtung zum Befüllen eines flexiblen Behälters
Dispositif de remplissage de réservoir flexible

(43) Date of publication of application: 08.12.2010
(73) Proprietor: F.Hoffmann-La Roche AG, 4070 Basel (CH); Roche Diagnostics GmbH, 68305 Mannheim (DE)
(72) Inventor: Kühni, Florian, 3400 Burgdorf (CH); Huwiler, Christoph, 6340 Baar (CH); Wyss, Martin, 3400 Burgdorf (CH)
(74) Representative: Rentsch Partner AG

(56) References cited:
- WO-A1-02/38957
- FR-E- 8 120
- US-A- 4 817 687
- US-A- 4 832 096
- US-A1- 2002 128 628

## Description

### Field of the Invention

The invention relates to devices for filling flexible containers for storing liquid medicaments, to be administered to a patient by an infusion pump device, and methods for filling flexible containers.

### State of the art

Devices for the automated release of liquid medicaments are normally used with patients who have a continuous and in the course of the day varying need of a medicine that can be administered by subcutaneous infusion. Specific applications are, for example, certain pain therapies and the treatment of diabetes, in which computer controlled infusion pump devices are used, such as insulin pumps. Such devices can be carried by a patient on the body, and contain a certain amount of liquid medicament in a medicine reservoir in the form of a container. The medicine reservoir often comprises medicine sufficient for one or several days. The liquid medicament is supplied to the patient's body from the medicine reservoir by subcutaneous infusion of injection, through an infusion cannula or an injection needle.

As used herein, the terms "medicament" and "liquid medicament" are meant to encompass any drug-containing flowable medicine, or therapeutic or diagnostic liquid, capable of being passed through a delivery element such as a hollow needle in a controlled manner, such as a liquid, solution, gel or fine suspension. Representative drugs include pharmaceuticals such as peptides, proteins, and hormones, biologically derived or active agents, hormonal and gene based agents, nutritional formulas and other substances in both solid (dispensed) or liquid form. In particular the term medicament encompasses insulin preparations ready for administration.

The terms "subcutaneous infusion" and "subcutaneous injection" are meant to encompass any method in which a needle device is inserted at a selected site within the body of a patient for subcutaneous, intravenous, intramuscular or intradermal delivery of a liquid medicament to a subject. Further, the term needle defines a piercing member (including an array of micro needles) adapted to be introduced into or through the skin of a subject.

Particularly in self-administration of liquid medicaments, for example insulin, the patients using the medicament in question and administering it themselves by means of an infusion pump are increasingly emphasizing convenience and discretion. As a consequence the dimensions of such infusion devices are limited, and particular the overall length, width and thickness should be as small as possible, in order not be evident through clothing and to be carried as comfortable as possible.

While there are fully or partly disposable single-use infusion pump devices, such devices are typically non-disposable and are loaded with a disposable drug cartridge. Disposable cartridges are preferable for sterility and contamination prevention reasons. They may be delivered pre-filled with a certain liquid medicament, or empty, ready to be filled by a user. Said self-filling of containers has the advantage that also medicaments that are not readily available in pre-filled containers can be used for such infusion pump devices, thereby providing the patient with a larger choice of sources for his medicaments. Furthermore the stability of many medicaments in liquid form, particularly in plastic containers, can only be guaranteed by the manufacturer for some days.

The standard infusion pump devices that are carried on or near the body have a medicine reservoir with a cylindrical ampoule and a displacement piston, which is pushed into the ampoule by a piston rod or threaded spindle in order to convey the liquid medicament. These known designs have the disadvantage of being longer and/or thicker than desired, the resulting dimensions being detrimental to the provision of compact infusion pumps.

Manufacturers try to meet the demand of small infusion pump devices by various means. For example may the cylindrical ampoule be replaced by a container with a rectangular or another suitable cross-section, interacting with a displacement piston of corresponding shape. Different embodiments of such compact medicine reservoir devices are shown in WO 2008/122135 A1.

A further approach to reduce the overall volume of an infusion pump device is to replace the syringe-type dosing mechanism, in which a piston is displaced along a long container axis by an actuator, thereby conveying the appropriate amount of liquid medicine, by a downstream pump system. In such a device a miniaturized pump is arranged downstream of the reservoir, and causes a suction pressure that conveys the product from the reservoir to its destination. An example for such a pump is WO 2004/009162 A1.

Preferably the reservoir in such a type of an infusion pump device is realized as a flexible container. Such a flexible container may for example have the form of two flexible wall sheets that are sealed together. Flexible containers have the advantage of a smaller volume surplus of the container in relation to its content, which reduces the manufacture costs and the achievable dimensions of an infusion pump device using such a flexible container. The volume of a flexible container for use in an infusion pump device may be up to 10 ml, for example. A typical range for diabetes therapy is 1.5 to 3.5 ml. For other therapies, e.g. pain therapy, which require other administration regimes, other volume ranges may be more preferable.

A common problem of flexible containers as they are known is air remaining in the container. If for example a flexible container is provided empty, and is filled with the appropriate liquid medicament by the user himself, there may be a certain amount of air in the container after the filling process. If said air remains in the container or in the fluidic system of a pump system, air bubbles may be administered instead of the liquid medicament, which leads to potentially dangerous dosing errors. Furthermore the administration of air into a patient's body should generally be avoided for medical reasons.

Yet another negative effect of air present in the fluidic system of an infusion pump device is the reduced stiffness of the fluidic system. Due to the high compressibility of gases such as air in relation to liquids such as water, it becomes difficult to measure the exact pressure in the fluidic system. This impedes the detection of blockages or occlusions in the fluidic system of an infusion pump device by measuring the fluidic pressure.

Filling a container with a liquid medicament with prior removal of air from the container and without having air bubbles getting into the container requires a considerable skill of a user. Devices are known from the state of the art that should help a user to fill a container for infusion pump systems. Said devices, however, are intended for the use in syringe/ampoule type infusion pump systems, and cannot be properly used with flexible containers. Such filling devices are disclosed, among others, in EP 1952837 A1 and EP 1820485 A1.

US 4817887 discloses a filling device for sterile filling of flexible containers. A flexible temporary storage container, fluidly connected to a liquid reservoir and the container to be filled, is placed in a dosage chamber. In a first step the dosage chamber is subjected to underpressure. The flexible temporary storage container expands against the walls of the dosage chamber. The temporary storage container, having underpressure, is fluidly connected to a liquid reservoir, and a portion of liquid is sucked into the temporary container. In a second step the filled temporary container is fluidly connected to the flexible container to be filled, which has been previously evacuated. The dosage chamber is subjected to overpressure, and as a result the liquid is conveyed from the temporary storage container to the flexible container.

FR 8120 discloses a device for filling cans with a liquid. In one state of the device, a rigid temporary storage container is subject to underpressure, thereby retrieving a portion of liquid from a reservoir. At the same time the can is evacuated. In a second state, the evacuated can is connected to normal atmospheric pressure via the temporary storage container, filled with liquid. As a result the portion of liquid is conveyed to the can driven by the pressure differential between can and atmosphere, and gravitation. If necessary the can be subjected to a further evacuation step, in order to complete the filling.

### Objects of the Invention

One object of the invention is to provide an advantageous device for filling a flexible liquid medicament container with a liquid from a liquid medicament reservoir. A device according to the invention should allow the efficient filling of a flexible container with a liquid medicament. The device should be easily operated by a user.

Another object of the invention is to provide an advantageous device for filling a flexible container with a liquid from a reservoir that allows the removal of air that is already present in the flexible container.

Furthermore such a device advantageously should prevent the entering of air bubbles into the flexible container.

A device according to the invention should be producible with high quality at low costs, and should comprise a minimum number of components.

Yet another object of the invention is to provide an advantageous method for filling a flexible container, particularly a flexible container for use in an infusion pump device, with a liquid from a reservoir.

These and other objects are achieved by device for filling a flexible reservoir, and a use of such a device for filling a flexible container, according to the independent claims. Advantageous embodiments are given in the dependent claims.

### Summary of the invention

To fill a flexible liquid medicament container, particularly a collapsible container, with a liquid, while at the same avoiding the presence of air in the container, the container is evacuated prior to filling it with the liquid. This measure significantly reduces the volume of air remaining in the container, including the air in the dead volume of said container, and facilitates an efficient filling of the container in a next step. The transfer of the liquid from a primary reservoir to the container is driven by a temporarily generated pressure difference between the primary reservoir and the container.

If a user would carry out the necessary steps himself, by using appropriate tools such as syringes, such a procedure would be rather complicated, since it involves a number of steps that have to be carried out in the correct order. Furthermore care has to be taken to avoid the transfer of air bubbles into the flexible container during the filling step.

A device according to the invention for filling a flexible liquid medicament container with a liquid from a liquid medicament reservoir comprises a pressurizing chamber, a displacement piston, being sealingly and slideably arranged in the pressurizing chamber, an evacuation chamber, and a displacement piston, being sealingly and slideably arranged in the evacuation chamber. Each chamber with its corresponding displacement piston defines an inner volume of the chamber. A pressurizing conduit is arranged to fluidly connect the inner volume of the pressurizing chamber with a reservoir adapter arranged to be connected to the liquid reservoir; an evacuation conduit is arranged to fluidly connect the inner volume of the evacuation chamber with a container adapter arranged to be connected with the flexible container; and a transfer conduit is arranged to fluidly connect the reservoir adapter with the container adapter. A valve, preferably a multi-way valve, is arranged to connect the container adapter either with the evacuation conduit or the transfer conduit.

Advantageously in such a device according to the invention the movements of the two displacement pistons are coupled. Preferably the movements of the two displacement pistons are coupled in such a way that when one displacement piston is shifted and decreases the inner volume of the corresponding chamber, the other displacement piston is also shifted and increases the inner volume of the corresponding other chamber, and vice versa.

A particularly preferred embodiment of a device according to the invention is designed in such a way that only one stroke of the displacement pistons is sufficient to fill the flexible container. This can be achieved by properly choosing the dimensions of the chambers and the pistons, and adapting the coupling mechanism between the pistons.

In an especially preferred embodiment the two chambers are realized as one closed cylinder, and the two displacement pistons are realized as one single displacement piston or as two rigidly connected displacement pistons, arranged in said cylinder and dividing it into the two distinct chambers. The one single displacement piston or the two rigidly connected displacement pistons are displaceable along a longitudinal axis of said cylinder.

Another advantageous embodiment of the device according to the invention comprises two subunits. A first subunit comprises the reservoir adapter, the container adapter and the transfer conduit; and a second subunit comprises the two chambers and displacement pistons. The first subunit is detachable from the second subunit.

In yet another embodiment of the device, the reservoir adapter and/or the container adapter for connection with the liquid reservoir are detachable from the rest of the device.

Preferably the reservoir adapter comprises two parallel hollow needles or pins that are arranged to penetrate a septum of a liquid reservoir in the form of a vial. A first needle or pin is fluidically connected to the pressurizing conduit, and a second needle or pin is fluidically connected to the evacuation conduit. More preferably the first needle or pin is substantially longer than the second needle or pin.

Advantageous embodiments of the device according to the invention comprise a degassing membrane, arranged to remove gas, particularly air, from the transfer conduit between the reservoir adapter and the container adapter. Additionally or alternatively a bubble trap or a bubble filter, and/or a check valve, and/or a capillary segment or flow restrictor is arranged in the transfer conduit between the reservoir adapter and the container adapter.

In yet another preferred embodiment of a device according to the invention, the pressurizing conduit and/or the evacuation conduit comprise a gate valve, arranged to temporarily interrupt the corresponding conduit, depending on the position of the displacement piston within the corresponding chamber.

In a preferred variant a conduit comprising such a gate valve is divided into two segments. A first segment is located in the structure of the device, and a second segment is located in the corresponding displacement piston. Depending on the position of the displacement piston within the corresponding chamber the cross-sections of the two segments either overlap and fluidly connect the two segments, or do not overlap, thereby implementing the gate valve. Advantageously in such an embodiment of a device the segment of the pressurizing and/or the evacuation conduit that is located within the displacement piston ends in an outlet located on the front face of the corresponding displacement piston, facing toward the inner volume.

Preferably, in a device according to the invention the displacement pistons are manually operated.

It is also preferable that the multi-way valve of a device according to the invention is actuated by a movement of one or both displacement pistons, or by the movement of an actuation mechanism or actuator for the displacement pistons, or by a locking mechanism for the displacement pistons.

Such devices according to the invention are particularly useful for filling a flexible container for use in an infusion pump device.

In an exemplary method according to the disclosure for filling a flexible container with a liquid from a reservoir, (i) the flexible container is evacuated; (ii) the liquid reservoir is pressurized; and (iii) the liquid is transferred from the liquid reservoir to the flexible container, driven by a pressure difference between the liquid reservoir and the flexible container. Step (i) is carried out before or after step (ii), or is carried out at least partially at the same time as step (ii).

In a preferred variant of such a method, the pressure in step (ii) is produced by decreasing the inner volume of a pressurizing chamber connected to the liquid reservoir. The negative pressure in step (i) can be produced by increasing the inner volume of an evacuation chamber connected to the flexible container.

Advantageously such a method is carried out with a device according to the invention as it has been discussed above.

### Brief description of the drawings

In order to facilitate a fuller understanding of the present invention, reference is now made to the appended drawings. These references should not be construed as limiting the present invention, but are intended to be exemplary only.
- Figure 1: schematically shows a device according to the invention for filling a flexible container.
- Figure 2: shows different possible variants of the mechanical coupling between the two displacement pistons in a device according to the invention.
- Figure 3: schematically shows a cross-section of an advantageous embodiment of device according to the invention for filling a flexible container.
- Figure 4: shows the device in Figure 1 in a perspective view.
- Figure 5: schematically explains a method according to the invention for filling a flexible container.
- Figure 6: schematically shows a cross-section of another embodiment of a device accord- ing to the invention.

### Description of embodiments of the invention

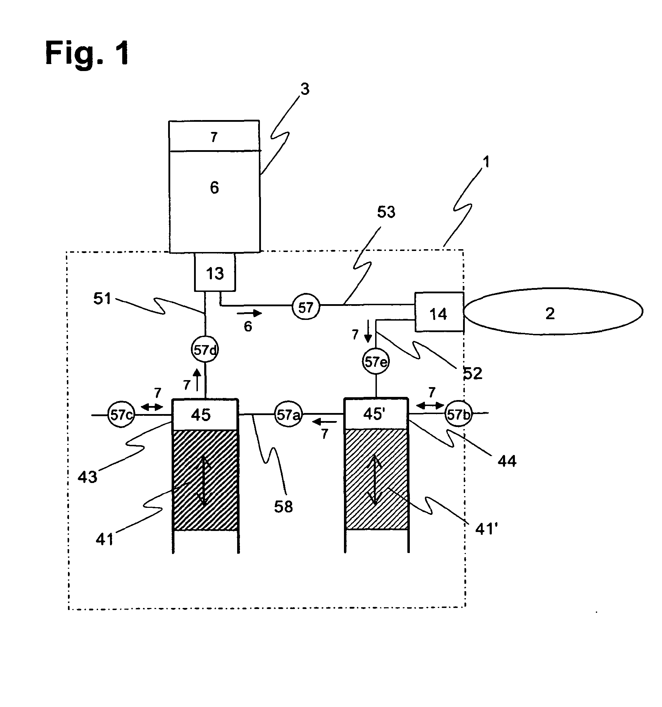

A device 1 according to the invention for filling a flexible container 2 is shown in Figure 1 in a generalized scheme. The device 1 comprises a pressurizing chamber 43, with a movable displacement piston 41 sealingly closing the chamber, and defining an inner volume 45 of the pressurizing chamber 43.

The inner volume 45 is fluidly connected via a pressurizing conduit 51 to an first adapter (reservoir adapter 13), to which a liquid reservoir 3 can be connected, for example a vial containing a liquid medicament. When the displacement piston 41 is moved into the pressurizing chamber 43, thereby decreasing its inner volume 45, the resulting increasing pressure is transferred from the pressurizing chamber 43 to the interior of the liquid reservoir 3.

The device 1 furthermore comprises an evacuation chamber 44, with a movable displacement piston 41' sealingly closing the chamber, and defining an inner volume 45'. Said inner volume 45' is fluidly connected via a conduit 52 to another adapter (container adapter 14), to which a flexible container 2, to be filled with the liquid 6 in the reservoir 3, can be connected. When the displacement piston 41' is moved out of the evacuation chamber 44, thereby increasing the inner volume 45, the pressure decreases, and the air in the flexible container 2 and/or the adapter 14 is sucked into the evacuation chamber 44. If necessary the flexible container 2 may even be deflated this way, prior to evacuation.

For transferring the liquid 6 from the reservoir 3 to the flexible container 2, the two adapters 13, 14 are fluidly connected via a transfer conduit 53. After evacuation of the flexible container 2, the evacuation conduit 52 is temporarily interrupted, for example by closing a valve 57e arranged in said conduit 52. Then the previously closed transfer conduit 53 is temporarily opened, for example by opening a valve 57 arranged in the transfer conduit 53. The liquid 6 then flows from the pressurized liquid reservoir 3 to the flexible container 2. A further valve 57d can optionally be mounted in the pressurizing conduit 51, for example for closing the conduit 51 after pressurizing the reservoir 3.

In another advantageous embodiment of the device, the two chambers 43, 44 are connected via a fourth conduit 58 with valve 57a, which can be used to partially compensate the pressure difference between the two chambers, or to transfer air from one chamber to the other, for example during a resetting step of the device 1, when the pistons 41, 41' are moved to their original positions. Further conduits with valves 57b, 57c can be provided, for controlling the pressure difference between the chambers 43, 44 and the outside.

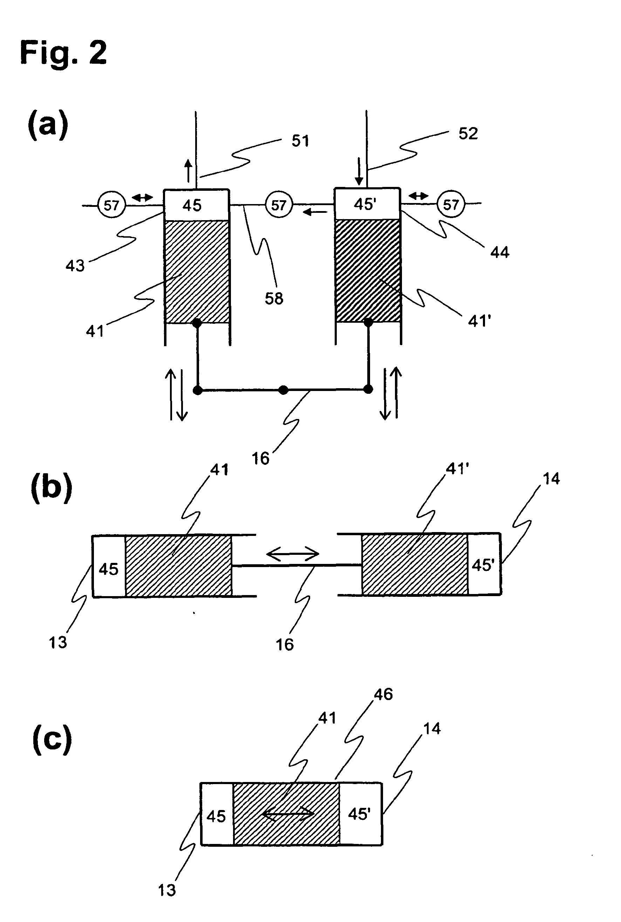

In an advantageous embodiment of a device 1 according to the invention, the movement of the two displacement pistons 41, 41' is mechanically coupled. Particularly the movement is mechanically coupled in such a way that a movement of one piston 41 , which increases respectively decreases the inner volume 45 of the corresponding chamber 43, is combined with a movement of the other piston 41', which decreases respectively increases the inner volume 45' of the other chamber 44, and vive versa. A number of embodiments of such a mechanical coupling 16 between the two displacement pistons 41, 41 ' is shown in Figure 2.

In Figure 2(a) for example, two parallel pistons 41, 41' are coupled by a pivot arm mechanism 16. Also possible is the use of a suitable crankshaft mechanism, although such a solution would be more complex. Another possible embodiment is shown in Figure 2(b), where two reversely aligned pistons 41, 41' are connected by a rigid rod 16.

In a particularly advantageous variant, as disclosed in Figure 2(c), the two chambers 13, 14 are part of one single chamber 46. The two pistons are combined to one single displacement piston 41, moveably arranged in said single chamber 46, and separating the chamber into two distinct inner volumes 45, 45'.

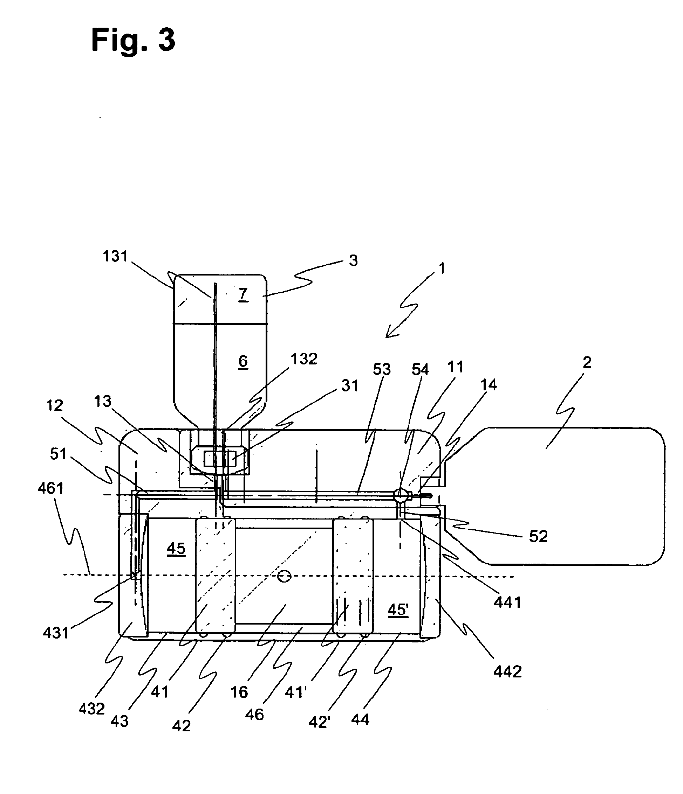

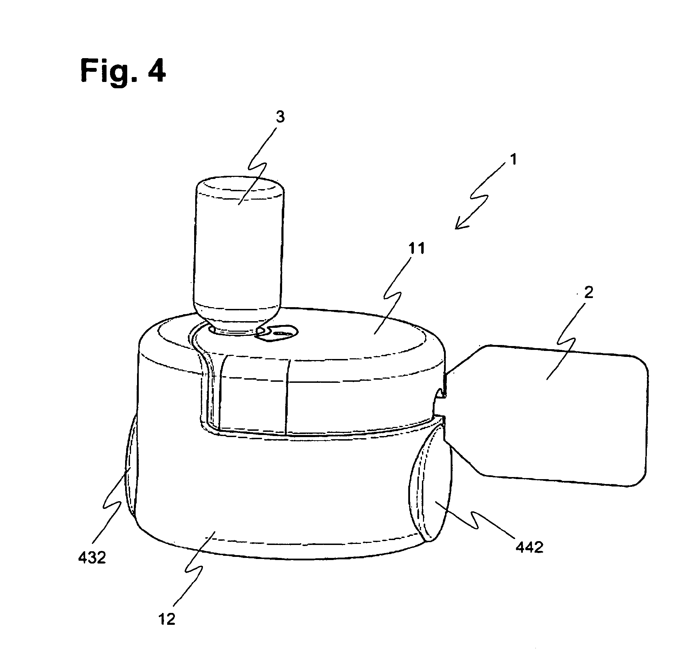

An advantageous embodiment of a device according to the invention for filling a flexible container is shown in Figure 3, in a cross-section, and in Figure 4, in a perspective view. In the shown embodiment of the device 1, the pressurizing chamber 43 and the evacuation chamber 44 are realized by a single cylinder 46, in which two short displacement pistons 41, 41' are arranged, connected by a rigid coupling piece 16. The two ends of the cylinder 46 are sealingly closed with two cover plates 432, 442. For each piston 41, 41', two circumferential sealings 42 provide an air-tight closure of the inner volumes 45, 45'. The combined displacement piston structure 41, 16, 41' is slideably movable along a longitudinal axis 461 of the cylinder 46.

A pressurizing conduit 51 located in the structure of the body of the device 1 leads from an inlet 431 on the concave front face of the pressurizing chamber 43 to the reservoir adapter 13, where it is fluidly connected to a hollow needle 131. A short evacuation conduit 52 connects an outlet 441 in the cylinder wall with a multi-way valve 54, which is also fluidly connected with the container adapter 14 and the transfer conduit 53. The transfer conduit leads from the multi-way valve 54 to the reservoir adapter 13, where it is fluidly connected with another hollow needle 132.

In Figure 3 and 4 a liquid reservoir 3, namely a reversely mounted vial, is mounted to the reservoir adapter 13 of the device 1. The two hollow needles or pins 131, 132 of the adapter 13 protrude through a septum 31 of the vial 3. The length of the first hollow needle 131, connected to the pressurizing conduit 51, is chosen such that its tip is near the bottom of the vial 3. The length of the second hollow needle 132, connected to the transfer conduit 53, is such that its tip is close to the septum 31. This embodiment has the advantage that air, pressed into the reservoir 3 during the pressurizing step, is not injected into the liquid and thus is less likely to form air bubbles. This decreases the probability of air bubbles entering the transfer conduit 53 and thus potentially the flexible container 2.

The container adapter 14 may be connected directly with the flexible container 2, or via disposable connection element 141.

The embodiment of a device 1 shown in Figure 3 and 4 comprises two subunits 11, 12 that can be detached from each other. A first, reusable subunit 12 comprises the two chambers 43, 45 and pistons 41, 41' and all parts that do not come into contact with the liquid 6 during normal and proper use of the device 1. There is thus no risk that said subunit 12 gets contaminated.

A second, disposable subunit 11 comprises the adapter reservoir 13 for the reservoir 3, the container adapter 14 for the flexible container 2, and the transfer conduit 53. All parts of the device 1 that come into contact with the liquid 6 to be transferred, and thus have to be regularly replaced for hygienic reasons, are located in this disposable subunit 11. All the more complex parts are located in the subunit 12, and can be used several times.

A further advantage of two subunits 11, 12 is the pressure equalization between the two chambers 43, 44 that automatically takes place when detaching one subunit. With detached disposable subunit 11 the pistons can easily be brought back into the starting position.

To prevent any liquid from unintentionally entering the pressurizing conduit 51 or the evacuation conduit 52, hydrophobic filters may be arranged in said conduits, preferably located in the disposable subunit 11. Another possibility is the use of non-return valves, particularly in the pressurizing conduit.

The rigid piston structure 41, 16, 41 can be actuated by any suitable mechanism. Since the space of the cylinder 46 between the two pistons 41, 41' does not necessarily have to be closed air-tight, the rigid coupling piece 16 is readily accessible for an external actuation mechanism or actuator. In a simple embodiment, for example, a lever or arm (not shown) protrudes from the outside into the cylinder 46, and is connected with the coupling piece 16. By actuating the lever by hand, the user can move the pistons 41, 41' along the longitudinal axis 461 of the cylinder 46.

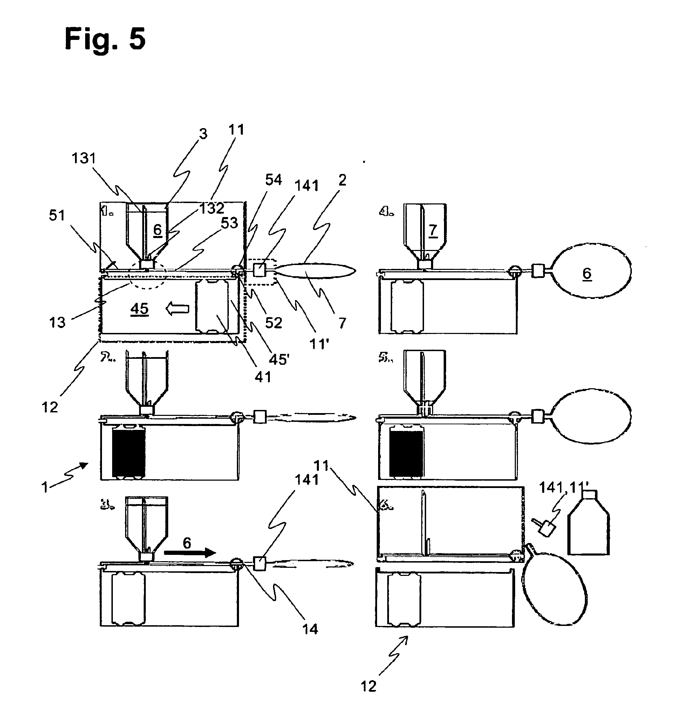

The method according to the invention for filling a flexible reservoir 2 is explained in Figure 5. Figure 5 also shows the normal use of an embodiment of a device 1 according to the invention as shown in Figures 3 and 4.

To carry out the filling process, the device 1 has to be properly prepared (step 1 in Figure 5). A vial 3 with a liquid medicament 6 is connected with the reservoir adapter 13. The two pistons, realized as a double piston 41, are in a position where the inner volume 45 of the pressurizing chamber 43 is maximum, and the inner volume 45' of the evacuation chamber 44 is minimum. The flexible container 2 is fluidly connected two a disposable connection element 141, which is connectable to the container adapter 14. The multi-way valve 54 is switched such that the transfer conduit 53 is interrupted (black marking), and the evacuation conduit 52 is fluidly connected to the flexible container 2. The flexible container 2 is not yet completely deflated, and contains some remaining air 7 inside.

In a next step (step 2 in Figure 5), the piston 41 is moved to the left, thereby increasing the inner volume 45' of the evacuation chamber 44 and decreasing the inner volume 45 of the pressurizing chamber 43. As a result, the remaining air inside of the container 2 is sucked into the evacuation chamber 44. The air in the flexible container 2 is thus almost completely removed, and the inner pressure in the flexible container is reduced to a minimum. Even the amount of air in the dead volume of the container is reduced, when calculated to normal pressure. At the same time the air in the inner volume 45 of the pressurizing chamber 43 is compressed, and the interior of the vial 3 is pressurized via the pressurizing conduit 51.

Subsequently, when the piston 41, 41' has reached a maximum position (step 3 in Figure 5) the valve 54 is switched such that that the evacuation conduit 52 is disconnected from the container adapter 14 and container 2, and the transfer conduit 53 is fluidly connected with the container 2. The liquid medicament can now flow via the transfer conduit 53 from the reservoir 3 to the container 2, driven by the pressure difference between the vial 3 (increased pressure) and the flexible container 2 (atmospheric pressure, as long as maximum filling capacity is not reached). To limit the maximum flow speed of the liquid during transfer, particularly during the beginning of the transfer, the transfer conduit 53 may be provided with a suitable means, for example meanders, flow restrictors or capillaries.

The switching of the valve may for example be carried out by a user, or may be triggered automatically, for example when the piston 41, 16, 41' reaches a certain position. It is also possible to provide a locking mechanism for the piston 41, 16, 41' at the maximum position. The locking of the piston can then be used as the trigger for switching the valve 54. Preferably the locking is releasable for reusing the device.

In another advantageous variant the locking may only be released when a used, disposable subunit has been removed. Such an embodiment has the advantage that the risk of a accidental contamination of the reusable subunit caused by an operating error of a user is further reduced.

The valve 54 remains in its current state until the desired filling level of the container 2 has been reached, or the vial 3 has been completely emptied (step 4 in Figure 5). In Figure 5 the volume of the vial 3 and the container 2 are assumed to be equal, for simplicity. In reality, however, the volume of the reservoir 3 will be considerably larger than the volume of the flexible container 2 to be filled, the content of the vial 3 thus being sufficient to fill several of the flexible containers 2. A typical insulin vial for example contains 10 ml of insulin solution, which may be sufficient to fill up three to five flexible containers for use in an insulin infusion pump device.

Now the valve 54 is switched again, and both the evacuation conduit 52 and the transfer conduit 53 are disconnected from the container adapter 14 (step 5 in Figure 5).

In a last step (step 6 in Figure 5) the filled flexible container 2 is detached from the connection element 141, and may then be connected to an infusion pump device. The disposable 11' connection element 141 is also removed from the adapter 14. In the shown example the vial 3 is also completely empty, and is removed from the adapter 13. The disposable subunit 11 is also removed from the reusable subunit 12 of the device 1.

In another variant of the invention the connection element 141 may remain connected to the flexible container 2, and may even be used for liquidly connecting the container with an infusion pump device.

The adapter 13 may comprise a mechanism that does prevent the removal of the vial 3 from the adapter 13, for example as long as the disposable subunit 11 is connected to the reusable subunit 12. The removal of the vial 3 may even be impossible at all after connecting it to the adapter 13, so that the vial 3 and the disposable subunit 11 have to be disposed together.

Prior to the next use, the piston 41 is set back to the starting position, and is moved to the right. For that purpose the locking mechanism of the piston may have to be released. A spring element may be used to move the piston back to the first position. A new disposable subunit 11 is then attached to the subunit 12, resulting in a fresh device according to the invention, ready for use. As an alternative to a spring, the pistons may be set back to the starting position by manually operating the actuation mechanism of the pistons, for example with a lever or the like.

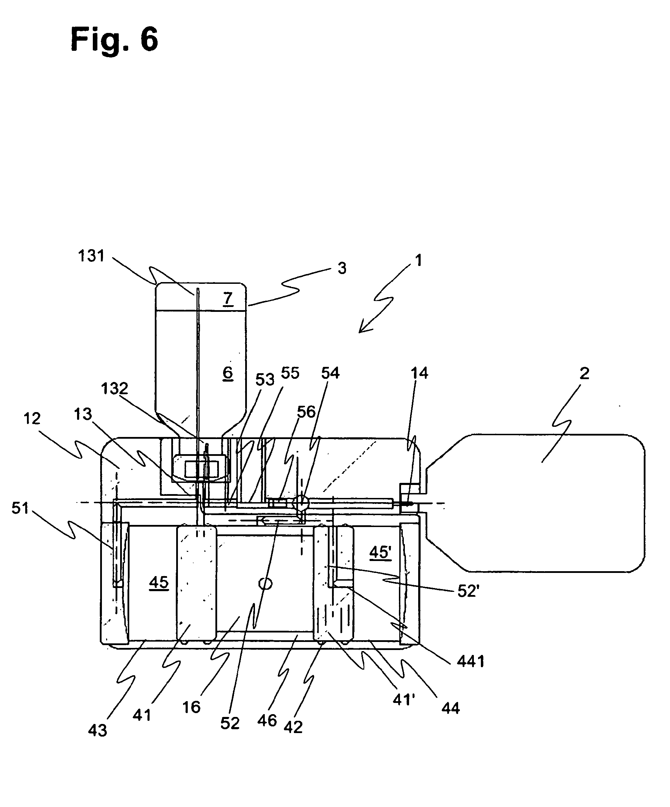

A further advantageous embodiment of a device 1 according to the invention is shown in Figure 6. The basic structure and composition is similar to the embodiment disclosed in Figure 3 and 4.

The multi-way valve 54 is shifted to the left, closer to the adapter 13. This has the advantage that the volume of the transfer conduit 53 is reduced, and thus also the potential amount of air remaining in said conduit.

A bubble trap 56 is arranged directly before the valve 54. Such a bubble trap or bubble filter is able to remove air bubbles from the liquid stream, or at least retain them in the trap. An advantageous type of bubble trap, particularly suitable for use in a device according to the invention, is for example disclosed in the European application No. 09155216 of the applicants. Said application is hereby incorporated by reference in its entirety as part of this disclosure.

Upstream of the bubble trap 56 a degassing membrane 55 is arranged in the wall of the conduit 53, in order to allow any air remaining in the conduit to permeate through the membrane 55, while any liquid 6 is retained in the conduit 53. Advantageously, after the pressurizing of the vial 3 a certain time period is allowed to pass prior to switching the valve 54 and connecting the transfer conduit 53 to the container 2. Driven by the pressure difference between vial and outside, the complete air in the transfer conduit will leave the conduit 53 through the degassing membrane and is replaced by the liquid 6.

In the embodiment in Figure 6 the evacuation conduit comprises two segments 52, 52'. A first segment 52 located in the structure of the device leads from the valve 54 to the wall of the cylinder 46. A second segment 52' is located in the piston 41', and leads from the outlet 441 located on the front face of the piston 41' to an opening located between the two sealings 42 of the piston 41'. The piston 41' and the evacuation conduit 52, 52' thus act as a gate valve, since the evacuation conduit is closed as long as the openings of the two segments 52, 52' do not overlap. As a consequence the air in the container 2 is not sucked into the evacuation chamber 44 continuously during the shifting of the pistons 41,41', but only during a later stage, when the pistons have come to the maximum position at the left. The two segments 52, 52' of the evacuation conduit are the fluidly connected, and the valve 54 has been switched to connect the evacuation conduit 52, 52' to the container 2. Such an embodiment thus allows separating in time the two functions of pressurizing the reservoir 3 and evacuating the flexible container 2, without needing an additional valve 57e in the evacuation conduit 52.

When configuring and designing a device according to the invention, care must be taken for the various pressure values in the different parts of the system. Said pressure values may also change during use, particularly between different filling rounds with the same vial. It is also possible to provide a device according to the invention with a mechanism to adapt the position of the pistons, in order to take into account varying process parameters, such as for example the filling level of the liquid reservoir. In typical embodiments, the liquid reservoir 3 (e.g. a vial) stores considerably more liquid medicine 6 than the flexible container 2, as has been mentioned above in the discussion of Figure 5.

While particular embodiments of the present invention have been illustrated and described, it would be obvious to those skilled in the art that various other changes and modifications can be made. The scope of the invention is therefore defined by the appended claims.

### List of Reference Numerals

- 1: device for filling a flexible container
- 11, 11': disposable subunit
- 12: reusable subunit
- 13: adapter for the liquid reservoir
- 131, 132: hollow needle or pin
- 14: adapter for the flexible container
- 141: connection element
- 16: mechanical coupling
- 2: flexible container
- 3: liquid reservoir, vial
- 31: septum
- 41, 41': displacement piston
- 42, 42': sealing
- 43: pressurizing chamber
- 431: inlet
- 432: cover plate
- 44: evacuation chamber
- 441: outlet
- 442: cover plate
- 45, 45': inner volume
- 46: cylinder
- 461: longitudinal axis
- 51, 51': pressurizing conduit
- 52, 52': evacuation conduit
- 53: transfer conduit
- 54: multi-way valve
- 55: degassing membrane
- 56: bubble trap, bubble filter
- 57, 57a, 57b, 57c, 57d, 57e: valve
- 58: fourth conduit
- 6: liquid

## Claims

1. A device (1) for filling a flexible liquid medicament container (2) with a liquid from a liquid medicament reservoir (3), with a pressurizing chamber (43), **characterized by** a displacement piston (41), being sealingly (42) and slideably arranged in the pressurizing chamber (43), an evacuation chamber (44), and a displacement piston (41'), being sealingly (42') and slideably arranged in the evacuation chamber (44); wherein
- each chamber (43, 44) with its corresponding displacement piston (41, 41') defines an inner volume (45, 45') of the chamber (43, 44);
- a pressurizing conduit (51) is arranged to fluidly connect the inner volume (45) of the pressurizing chamber (43) with a reservoir adapter (13) arranged to be connected to the liquid reservoir (3);
- an evacuation conduit (52, 52') is arranged to fluidly connect the inner volume (45') of the evacuation chamber (44) with a container adapter (14) arranged to be connected with the flexible container (2);
- a transfer conduit (53) is arranged to fluidly connect the reservoir adapter (13) with the container adapter (14); and
- a valve (54), preferably a multi-way valve, is arranged to fluidly connect the container adapter (14) either with the evacuation conduit (52, 52') or the transfer conduit (53).

2. The device according to claim 1, **characterized in that** the movements of the two displacement pistons (41, 41') are coupled.

3. The device according to claim 1, **characterized in that** the movements of the two displacement pistons (41, 41') are coupled in such a way that when one displacement piston (41,41') is shifted and decreases the inner volume (45, 45') of the corresponding chamber (43, 44), the other displacement piston (41', 41) is also shifted and increases the inner volume (45', 45) of the corresponding other chamber (44, 43), and vice versa.

4. The device according to any of the preceding claims, **characterized in that** the device (1) is designed in such a way that only one stroke of the displacement pistons (41, 41') is sufficient to fill the flexible container (2).

5. The device according to any of the preceding claims, **characterized in that** the two chambers (43, 44) are realized as one closed cylinder (46), and that the two displacement pistons (41, 41') are realized as one single displacement piston (41) or as two rigidly connected (16) displacement pistons (41, 41'), arranged in said cylinder (46) and dividing said cylinder (46) into the two distinct chambers (43, 44), wherein the one single displacement piston (41) or the two rigidly connected (16) displacement pistons (41, 41') are displaceable along a longitudinal axis (461) of said cylinder (46).

6. The device according to any of the preceding claims, **characterized in that** the device comprises two subunits (11, 12), a first subunit (11) comprising the reservoir adapter (13), the container adapter (14), and the transfer conduit (53); and a second subunit (12) comprising the two chambers (43, 44) and displacement pistons (41, 41'); wherein the first subunit (11) is detachable from the second subunit (12).

7. The device according to any of the preceding claims, **characterized in that** the reservoir adapter (13) comprises two parallel hollow needles or pins (131, 132) that are arranged to penetrate a septum (31) of a liquid reservoir in the form of a vial (3), wherein a first needle (131) or pin is fluidically connected to the pressurizing conduit (51), and a second needle (132) or pin is fluidically connected to the evacuation conduit (52).

8. The device according to claim 7, **characterized in that** the first needle (131) or pin is substantially longer than the second needle (132) or pin.

9. The device according to any of the preceding claims, **characterized by** a degassing membrane (55) arranged to remove gas, particularly air, from the transfer conduit (53), and/or a bubble trap or a bubble filter (56) arranged in the transfer conduit (53) between the reservoir adapter (13) and the container adapter (14).

10. The device according to any of the preceding claims, **characterized by** a check valve (57) and/or capillary segment and/or flow restrictor arranged in the transfer conduit (53) between the reservoir adapter (13) and the container adapter (14).

11. The device according to any of the preceding claims, **characterized in that** the pressurizing conduit (51) and/or the evacuation conduit (52) comprise a gate valve, arranged to temporarily interrupt the corresponding conduit (51, 52), depending on the position of the displacement piston (41, 41') within the corresponding chamber (43, 44).

12. The device according to any of the preceding claims, **characterized in that** the displacement pistons (41, 41') are manually operated.

13. The device according to any of the preceding claims, **characterized in that** the multi-way valve (54) is actuated by a movement of one or both displacement pistons (41, 41'), or by the movement of an actuation mechanism or actuator for the displacement pistons (41, 41'), or by a locking mechanism for the displacement pistons (41, 41').

14. The device according to any of claims 1 to 13, for use in filling a flexible container (2) of an infusion pump device.

15. Use of a device according to any of claims 1 to 13 in a method for filling a flexible container (2) with a liquid (6) from a reservoir (3), **characterized in that** (i) the flexible container (2) is evacuated; (ii) the liquid reservoir (3) is pressurized; and (iii) the liquid (6) is transferred from the liquid reservoir (3) to the flexible container (2), driven by a pressure difference between the liquid reservoir (3) and the flexible container (2); wherein step (i) is carried out before or after step (ii), or is carried out at least partially at the same time as step (ii).

16. The use according to claim 15, **characterized in that** the pressure in step (ii) is produced by decreasing the inner volume (45) of a pressurizing chamber (43) connected (51, 13, 131) to the liquid reservoir (3), and/or the negative pressure in step (i) is produced by increasing the inner volume (45') of a evacuation chamber (44) connected (52, 14) to the flexible container (2).

## Patentansprüche

1. Eine Vorrichtung (1) zum Füllen eines flexiblen Flüssigmedikamentbehälters (2) mit einer Flüssigkeit aus einem Flüssigmedikamentreservoir (3), mit einer Druckbeaufschlagungskammer (43), **gekennzeichnet durch** einen Verdrängerkolben (41), der abdichtend (42) und verschiebbar in der Druckbeaufschlagungskammer (43) angeordnet ist, eine Evakuierungskammer (44) und einen Verdrängerkolben (41'), der abdichtend (42') und verschiebbar in der Evakuierungskammer (44) angeordnet ist; wobei
- jede Kammer (43, 44) mit ihrem entsprechenden Verdrängerkolben (41, 41') ein Innenvolumen (45, 45') der Kammer (43, 44) definiert;
- eine Druckbeaufschlagungsleitung (51), die dazu eingerichtet ist, das Innenvolumen (45) der Druckbeaufschlagungskammer (43) mit einem Reservoiradapter (13), welcher dazu eingerichtet ist, mit dem Flüssigkeitsreservoir (3) verbunden zu werden, in Fluidverbindung zu bringen;
- eine Evakuierungsleitung (52, 52'), die dazu eingerichtet ist, das Innenvolumen (45') der Evakuierungskammer (44) mit einem Behälteradapter (14), welcher dazu eingerichtet ist, mit dem flexiblen Behälter (2) verbunden zu werden, in Fluidverbindung zu bringen;
- eine Transferleitung (53), die dazu eingerichtet ist, den Reservoiradapter (13) mit dem Behälteradapter (14) in Fluidverbindung zu bringen; und
- ein Ventil (54), vorzugsweise ein Mehrwegeventil, , das dazu eingerichtet ist, den Behälteradapter (14) entweder mit der Evakuierungsleitung (52, 52') oder der Transferleitung (53) in Fluidverbindung zu bringen.

2. Die Vorrichtung nach Anspruch 1, **dadurch gekennzeichnet, dass** die Bewegungen der zwei Verdrängerkolben (41, 41') gekoppelt sind.

3. Die Vorrichtung nach Anspruch 1, **dadurch gekennzeichnet, dass** die Bewegungen der zwei Verdrängerkolben (41, 41') derart gekoppelt sind, dass, wenn ein Verdrängerkolben (41, 41') verschoben wird und das Innenvolumen (45, 45') der entsprechenden Kammer (43, 44) verringert, der andere Verdrängerkolben (41', 41) ebenfalls verschoben wird und das Innenvolumen (45', 45) der entsprechenden anderen Kammer (44, 43) vergrößert, und umgekehrt.

4. Die Vorrichtung nach einem der vorstehenden Ansprüche, **dadurch gekennzeichnet, dass** die Vorrichtung (1) derart ausgestaltet ist, dass nur ein Hub der Verdrängerkolben (41, 41') ausreicht, um den flexiblen Behälter (2) zu füllen.

5. Die Vorrichtung nach einem der vorstehenden Ansprüche, **dadurch gekennzeichnet, dass** die zwei Kammern (43, 44) als ein geschlossener Zylinder (46) realisiert sind und dass die zwei Verdrängerkolben (41, 41') als ein einziger Verdrängerkolben (41) oder als zwei starr verbundene (16) Verdrängerkolben (41, 41') realisiert sind, die in dem Zylinder (46) angeordnet sind und den Zylinder (46) in die zwei verschiedenen Kammern (43, 44) aufteilen, wobei der eine einzige Verdrängerkolben (41) oder die zwei starr verbundenen (16) Verdrängerkolben (41, 41') entlang einer Längsachse (461) des Zylinders (46) verschiebbar sind.

6. Die Vorrichtung nach einem der vorstehenden Ansprüche, **dadurch gekennzeichnet, dass** die Vorrichtung zwei Untereinheiten (11, 12) umfasst, eine erste Untereinheit (11), umfassend den Reservoiradapter (13), den Behälteradapter (14) und die Transferleitung (53); und eine zweite Untereinheit (12), umfassend die zwei Kammern (43, 44) und Verdrängerkolben (41, 41'); wobei die erste Untereinheit (11) von der zweiten Untereinheit (12) abnehmbar ist.

7. Die Vorrichtung nach einem der vorstehenden Ansprüche, **dadurch gekennzeichnet, dass** der Reservoiradapter (13) zwei parallele hohle Nadeln oder Dorne (131, 132) umfasst, die angeordnet sind, ein Septum (31) eines Flüssigkeitsreservoirs in Form einer Ampulle (3) zu durchstechen, wobei eine erste Nadel (131) oder Dorn mit der Druckbeaufschlagungsleitung (51) in Fluidverbindung ist und eine zweite Nadel (132) oder Dorn mit der Evakuierungsleitung (52) in Fluidverbindung ist.

8. Die Vorrichtung nach Anspruch 7, **dadurch gekennzeichnet, dass** die erste Nadel (131) oder der erste Dorn wesentlich länger als die zweite Nadel (132) oder der zweite Dorn ist.

9. Die Vorrichtung nach einem der vorstehenden Ansprüche, **gekennzeichnet durch** eine Entgasungsmembran (55), die angeordnet ist, Gas, insbesondere Luft, aus der Transferleitung (53) zu entfernen, und/oder eine Blasenfalle oder einen Blasenfilter (56), angeordnet in der Transferleitung (53) zwischen dem Reservoiradapter (13) und dem Behälteradapter (14).

10. Die Vorrichtung nach einem der vorstehenden Ansprüche, **gekennzeichnet durch** ein Rückschlagventil (57) und/oder Kapillarsegment und/oder Durchflussdrossel, angeordnet in der Transferleitung (53) zwischen dem Reservoiradapter (13) und dem Behälteradapter (14).

11. Die Vorrichtung nach einem der vorstehenden Ansprüche, **dadurch gekennzeichnet, dass** die Druckbeaufschlagungsleitung (51) und/oder die Evakuierungsleitung (52) ein Absperrventil umfassen, das angeordnet ist, vorübergehend die entsprechende Leitung (51, 52) in Abhängigkeit von der Position der Verdrängerkolben (41, 41') in der entsprechenden Kammer (43, 44) zu unterbrechen.

12. Die Vorrichtung nach einem der vorstehenden Ansprüche, **dadurch gekennzeichnet, dass** die Verdrängerkolben (41, 41') manuell betätigt werden.

13. Die Vorrichtung nach einem der vorstehenden Ansprüche, **dadurch gekennzeichnet, dass** das Mehrwegeventil (54) durch eine Bewegung eines oder beider Verdrängerkolben (41, 41') oder durch die Bewegung eines Betätigungsmechanismus oder Stellglieds für die Verdrängerkolben (41, 41') oder durch einen Verriegelungsmechanismus für die Verdrängerkolben (41, 41') betätigt wird.

14. Die Vorrichtung nach einem der Ansprüche 1 bis 13, zur Verwendung beim Füllen eines flexiblen Behälters (2) einer Infusionspumpenvorrichtung.

15. Verwendung einer Vorrichtung nach einem der Ansprüche 1 bis 13 bei einem Verfahren zum Füllen eines flexiblen Behälters (2) mit einer Flüssigkeit (6) aus einem Reservoir (3), **dadurch gekennzeichnet, dass** (i) der flexible Behälter (2) evakuiert wird; (ii) das Flüssigkeitsreservoir (3) druckbeaufschlagt wird; und (iii) die Flüssigkeit (6) aus dem Flüssigkeitsreservoir (3) in den flexiblen Behälter (2), durch einen Druckunterschied zwischen dem Flüssigkeitsreservoir (3) und dem flexiblen Behälter (2) angetrieben, transferiert wird; wobei Schritt (i) vor oder nach Schritt (ii) durchgeführt wird, oder mindestens zum Teil gleichzeitig wie Schritt (ii) durchgeführt wird.

16. Die Verwendung nach Anspruch 15, **dadurch gekennzeichnet, dass** der Druck in Schritt (ii) durch Verringern des Innenvolumens (45) einer Druckbeaufschlagungskammer (43) erzeugt wird, die mit dem Flüssigkeitsreservoir (3) verbunden (51, 13, 131) ist, und/oder der Unterdruck in Schritt (i) durch Vergrößern des Innenvolumens (45') einer Evakuierungskammer (44) erzeugt wird, die mit dem flexiblen Behälter (2) verbunden (52, 14) ist.

## Revendications

1. Dispositif (1) de remplissage d'un contenant de médicament liquide souple (2) avec un liquide provenant d'un réservoir de médicament liquide (3), ayant une chambre de mise sous pression (43), **caractérisé par** un piston de déplacement (41), qui est agencé de manière étanche (42) et coulissante dans la chambre de mise sous pression (43), une chambre de production de vide (44) et un piston de déplacement (41') qui est agencé de manière étanche (42') et coulissante dans la chambre de production de vide (44) ; dans lequel
- chaque chambre (43, 44) avec son piston de déplacement correspondant (41, 41') définit un volume interne (45, 45') de la chambre (43, 44) ;
- un conduit de mise sous pression (51) est agencé pour relier fluidiquement le volume interne (45) de la chambre de mise sous pression (43) à un adaptateur de réservoir (13) agencé pour être relié au réservoir de liquide (3) ;
- un conduit de production de vide (52, 52') est agencé pour relier fluidiquement le volume interne (45') de la chambre de production de vide (44) à un adaptateur de contenant (14) agencé pour être relié au contenant souple (2) ;
- un conduit de transfert (53) est agencé pour relier fluidiquement l'adaptateur de réservoir (13) à l'adaptateur de contenant (14) ; et
- une vanne (54), de préférence une vanne multivoies, est agencée pour relier fluidiquement l'adaptateur de contenant (14) soit au conduit de production de vide (52, 52'), soit au conduit de transfert (53).

2. Dispositif selon la revendication 1, **caractérisé par le fait que** les déplacements des deux pistons de déplacement (41, 41') sont couplés.

3. Dispositif selon la revendication 1, **caractérisé par le fait que** les déplacements des deux pistons de déplacement (41, 41') sont couplés de telle sorte que, lorsqu'un piston de déplacement (41, 41') est décalé et diminue le volume interne (45, 45') de la chambre correspondante (43, 44), l'autre piston de déplacement (41', 41) est également décalé et augmente le volume interne (45', 45) de l'autre chambre correspondante (44, 43), et inversement.

4. Dispositif selon l'une quelconque des revendications précédentes, **caractérisé par le fait que** le dispositif (1) est conçu de telle sorte qu'une seule course des pistons de déplacement (41, 41') est suffisante pour remplir le contenant souple (2).

5. Dispositif selon l'une quelconque des revendications précédentes, **caractérisé par le fait que** les deux chambres (43, 44) sont réalisées sous la forme d'un cylindre fermé (46), et **par le fait que** les deux pistons de déplacement (41, 41') sont réalisés sous la forme d'un seul piston de déplacement (41) ou sous la forme de deux pistons de déplacement (41, 41') reliés rigidement (16), agencés dans ledit cylindre (46) et divisant ledit cylindre (46) en les deux chambres distinctes (43, 44), l'unique piston de déplacement (41) ou les deux pistons de déplacement (41, 41') reliés rigidement (16) étant aptes à se déplacer le long d'un axe longitudinal (461) dudit cylindre (46).

6. Dispositif selon l'une quelconque des revendications précédentes, **caractérisé par le fait que** le dispositif comprend deux sous-unités (11, 12), une première sous-unité (11) comprenant l'adaptateur de réservoir (13), l'adaptateur de contenant (14) et le conduit de transfert (53) ; et une seconde sous-unité (12) comprenant les deux chambres (43, 44) et les pistons de déplacement (41, 41'); la première sous-unité (11) étant détachable de la seconde sous-unité (12).

7. Dispositif selon l'une quelconque des revendications précédentes, **caractérisé par le fait que** l'adaptateur de réservoir (13) comprend deux aiguilles ou broches creuses parallèles (131, 132) qui sont agencées pour pénétrer dans une cloison (31) d'un réservoir de liquide se présentant sous la forme d'une ampoule (3), une première aiguille (131) ou broche étant reliée fluidiquement au conduit de mise sous pression (51) et une seconde aiguille (132) ou broche étant reliée fluidiquement au conduit de production de vide (52).

8. Dispositif selon la revendication 7, **caractérisé par le fait que** la première aiguille (131) ou broche est sensiblement plus longue que la seconde aiguille (132) ou broche.

9. Dispositif selon l'une quelconque des revendications précédentes, **caractérisé par** une membrane de dégazage (55) agencée pour retirer le gaz, en particulier l'air, du conduit de transfert (53), et/ou une trappe à bulles ou un filtre à bulles (56) agencé dans le conduit de transfert (53) entre l'adaptateur de réservoir (13) et l'adaptateur de contenant (14).

10. Dispositif selon l'une quelconque des revendications précédentes, **caractérisé par** un clapet de retenue (57) et/ou un segment capillaire et/ou un réducteur de débit agencés dans le conduit de transfert (53) entre l'adaptateur de réservoir (13) et l'adaptateur de contenant (14).

11. Dispositif selon l'une quelconque des revendications précédentes, **caractérisé par le fait que** le conduit de mise sous pression (51) et/ou le conduit de production de vide (52) comprennent un robinet de sectionnement, agencé pour interrompre temporairement le conduit correspondant (51, 52), en fonction de la position du piston de déplacement (41, 41') à l'intérieur de la chambre correspondante (43, 44).

12. Dispositif selon l'une quelconque des revendications précédentes, **caractérisé par le fait que** les pistons de déplacement (41, 41') sont actionnés manuellement.

13. Dispositif selon l'une quelconque des revendications précédentes, **caractérisé par le fait que** la vanne multivoies (54) est actionnée par un déplacement de l'un ou des deux pistons de déplacement (41, 41'), ou par le déplacement d'un mécanisme d'actionnement ou d'un actionneur pour les pistons de déplacement (41, 41'), ou par un mécanisme de verrouillage pour les pistons de déplacement (41, 41').

14. Dispositif selon l'une quelconque des revendications 1 à 13, destiné à être utilisé dans le remplissage d'un contenant souple (2) d'un dispositif de pompe à perfusion.

15. Utilisation d'un dispositif selon l'une quelconque des revendications 1 à 13 dans un procédé de remplissage d'un contenant souple (2) avec un liquide (6) provenant d'un réservoir (3), **caractérisée par le fait que** (i) le contenant souple (2) est mis sous vide ; (ii) le réservoir de liquide (3) est mis sous pression ; et (iii) le liquide (6) est transféré du réservoir de liquide (3) au contenant souple (2), entraîné par une différence de pression entre le réservoir de liquide (3) et le contenant souple (2) ; l'étape (i) étant réalisée avant ou après l'étape (ii), ou étant réalisée au moins partiellement en même temps que l'étape (ii).

16. Utilisation selon la revendication 15, **caractérisée par le fait que** la pression dans l'étape (ii) est produite par diminution du volume interne (45) d'une chambre de mise sous pression (43) reliée (51, 13, 131) au réservoir de liquide (3), et/ou la pression négative dans l'étape (i) est produite par augmentation du volume interne (45') d'une chambre de production de vide (44) reliée (52, 14) au contenant souple (2).
